(19) Europäisches Patentamt
European Patent Office
Office européen des brevets

(11) Publication number : **0 325 460 B1**

(12) # EUROPEAN PATENT SPECIFICATION

(45) Date of publication of patent specification : **21.10.92 Bulletin 92/43**

(21) Application number : **89300510.8**

(22) Date of filing : **19.01.89**

(51) Int. Cl.$^5$ : **C07D 487/04,** A61K 31/505, // (C07D487/04, 249:00, 239:00)

(54) **Dideoxydidehydrocarbocyclic nucleosides.**

(30) Priority : **20.01.88 US 146252**
**05.12.88 US 278652**

(43) Date of publication of application : **26.07.89 Bulletin 89/30**

(45) Publication of the grant of the patent : **21.10.92 Bulletin 92/43**

(84) Designated Contracting States : **AT BE CH DE ES FR GB GR IT LI LU NL SE**

(56) References cited :
**EP-A- 0 215 759**
**EP-A- 0 236 935**
**US-A- 4 268 672**
**JOURNAL OF ORGANIC CHEMISTRY vol. 52, 1987, pages 1344-1347; V. NAIR et al.: "2-halogenated purine nucleosides: synthesis andreactivity"**

(73) Proprietor : **REGENTS OF THE UNIVERSITY OF MINNESOTA**
**Morrill Hall, 100 Church Street Southeast**
**Minneapolis, Minnesota 55455 (US)**

(72) Inventor : **Vince, Robert**
**782 Hilltop Road**
**St Paul, Minnesota 55118 (US)**
Inventor : **Hua, Mei**
**Jla 35 Hua Yan Bei Road, 9-532**
**Haidian District, Beijing (CN)**

(74) Representative : **Holmes, Michael John et al**
**Frank B. Dehn & Co. Imperial House 15-19 Kingsway**
**London WC2B 6UZ (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to dideoxydidehydrocarbocyclic nucleoside analogues. More specifically, it is concerned with carbocyclic 2′,3′- dideoxy-2′,3′-didehydro-8-azapurine nucleoside analogues and their use in therapy, in particular as anticancer agents.

8-azapurine nucleosides are generally known in the art to exhibit antiviral activity. We have now found a novel class of 8-azapurine carbocyclic nucleosides having anticancer activity. There is accordingly provided in a first aspect of the present invention compounds of formula (I)

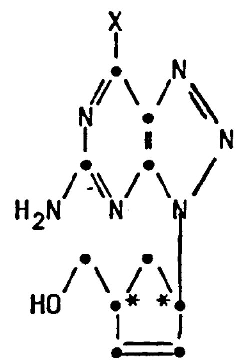

(I)

wherein X is hydrogen, $NRR^1$, SR, OR or halogen;

R and $R^1$ may be the same or different and are selected from hydrogen, $C_{1-4}$alkyl and aryl;

and pharmaceutically acceptable derivatives thereof.

It will be appreciated by those skilled in the art that the compounds of formula (I) are cis compounds and further that the compounds of formula (I) contain two chiral centres (shown in formula (I) by *) and may thus exist in the form of two optical isomers and mixtures thereof including racemic mixtures. All such isomers and mixtures thereof including racemic mixtures are included within the scope of the invention. Conveniently the compounds will be in the form of a racemic mixture.

It will also be appreciated by those skilled in the art that certain of the compounds of formula (I) may exist as a number of tautomeric forms and all such tautomers are included within the scope of the invention.

As used herein the term halogen refers to fluorine, chlorine, bromine and iodine; when X is halogen it is preferably chlorine.

As used herein $C_{1-4}$alkyl refers to a straight or branched chain alkyl group for example methyl, ethyl, n-propyl, iso-propyl, n-butyl, sec-butyl and t-butyl. Conveniently $C_{1-4}$alkyl is methyl.

As used herein aryl refers to any mono- or polycyclic aromatic moiety and includes unsubstituted and substituted aryl (such as phenyl, tolyl, xylyl, anisyl) and unsubstituted and substituted aralkyl including ar ($C_{1-4}$) alkyl such as phen($C_{1-4}$)alkyl for example benzyl, phenethyl.

A preferred group of compounds of formula (I) are those in which X represents $NRR^1$, OR or halogen,and R and $R^1$ are as defined above especially hydrogen.

When X represents $NNR^1$ X is preferably $NH_2$.

When X represents OR X is preferably OH.

When X represents halogen X is preferably chlorine.

Particularly preferred compounds of formula (I) are those in which X represents OH or chlorine.

By "a pharmaceutically acceptable derivative" is meant any pharmaceutically acceptable salt, ester, or salt of such ester, of a compound of formula (I) or any other compound which, upon administration to the recipient, is capable of providing (directly or indirectly) a compound of formula (I) or an active metabolite or residue thereof.

Preferred esters of the compounds of formula (I) include carboxylic acid esters in which the non-carbonyl moiety of the ester grouping is selected from hydrogen, straight or branched chain alkyl (e.g. methyl, ethyl, n-propyl, t-butyl, n-butyl), alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); amino acid esters (e.g. L-valyl or L-isoleucyl) and mono-, di- or tri-phosphate esters.

With regard to the above described esters, unless otherwise specified, any alkyl moiety present advantageously contains 1 to 18 carbon atoms, particularly 1 to 4 carbon atoms. Any aryl moiety present in such esters

advantageously comprises a phenyl group.

Pharmaceutically acceptable salts of the compounds of formula (I) include those derived from pharmaceutically acceptable inorganic and organic acids and bases. Examples of suitable acids include hydrochloric, hydrobromic, sulphuric, nitric, perchloric, fumaric, maleic, phosphoric, glycollic, lactic, salicyclic, succinic, toluene-p-sulphonic, tartaric, acetic, citric, methanesulphonic, formic, benzoic, malonic, naphthalene-2-sulphonic and benzenesulphonic acids. Other acids such as oxalic, while not in themselves pharmaceutically acceptable, may be useful in the preparation of salts useful as intermediates in obtaining the compounds of the invention and their pharmaceutically acceptable acid addition salts.

Salts derived from appropriate bases include alkali metal (e.g. sodium), alkaline earth metal (e.g. magnesium), ammonium and $NR_4^+$ (where R is $C_{1-4}$alkyl) salts.

References hereinafter to a compound according to the invention includes both compounds of formula (I) and their pharmaceutically acceptable derivatives.

Compounds of the invention possess anticancer activity and may be of particular benefit in the treatment of tumours including leukemias and solid tumours such as carcinomas, sarcomas and hepatomas. Thus, for example, compounds of the invention have been found to be cytotoxic to cultured P-388 leukemia cells.

There is thus provided as a further aspect of the invention a compound of formula (I) or a pharmaceutically acceptable derivative thereof for use as an active therapeutic agent in particular as an anticancer agent, for example in the treatment of the human or non-human animal body to combat cancers, particularly tumours therein.

In a further or alternative aspect there is provided a method for the treatment of the human or non-human animal body to combat cancers, particularly tumours, therein, which method comprises administering to the said body an effective amount of a compound of formula (I) or a pharmaceutically acceptable derivative thereof.

There is also provided in a further or alternative aspect use of a compound of formula (I) or a pharmaceutically acceptable derivative thereof for the manufacture of a medicament for the treatment of the human or non-human animal body to combat cancers, particularly tumours, therein.

Certain of the compounds of formula (I) are also useful as intermediates in the preparation of other compounds of the invention.

It will be appreciated that the compounds according to the invention may advantageously be used in conjunction with one or more other therapeutic agents, such as for example anti-emetic agents, immunosuppressive agents or different anti-cancer agents. It is to be understood that the present invention covers the use of a compound of formula (I) or a physiologically acceptable salt thereof in combination with one or more other therapeutic agents.

In a further aspect of the present invention we provide a pharmaceutical composition comprising as an active ingredient a compound of formula (I) or a physiologically acceptable derivative thereof together with one or more pharmaceutical carriers or excipients.

The compounds according to the invention may be formulated for administration in any convenient way, and the invention therefore also includes within its scope pharmaceutical compositions comprising a compound of formula (I) or a physiologically acceptable derivative thereof adapted for use in human or veterinary medicine. Such compositions may be presented for use in conventional manner in admixture with one or more physiologically acceptable carriers or excipients. The compositions may optionally further contain one or more other therapeutic agents such as a different anti-cancer agent or an anti-emetic agent or an immunosuppressive agent.

Thus, the compounds according to the invention may be formulated for oral, buccal, topical, rectal or parenteral administration (eg by bolus injection or intravenous infusion).

Injections are sterile products and may be presented in unit dose form, for instance as ampoules, vials, small volume infusions or pre-filled syringes, or in multi-dose containers, for example, with an added preservative. The compositions may take such forms as solutions, suspensions or emulsions in aqueous or non-aqueous vehicles, and may contain formulatory agents such as anti-oxidants, buffers, bulking agents, chelating agents, antimicrobial agents, solubilising agents, surfactants and/or tonicity adjusting agents. Alternatively, the active ingredient (with our without added substances) may be in a dry form for constitution with a suitable vehicle, eg sterile pyrogen-free water or dextrose, before use. The dry solid presentation may be prepared by filling a sterile powder aseptically into individual sterile containers or by filling a sterile solution aseptically into each container and freeze-drying.

The compounds according to the invention may also be formulated as compositions for oral administration. As tablets or capsules, they may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or

elixirs, or may be presented as a dry product for constitution with water or other suitable vehicle before use. Such liquid preparations may contain conventional additives such as suspending agents, surfactants, non-aqueous vehicles, preservatives, sugars, sweetening agents, buffers,colours, antioxidants and/or flavours. The compounds may also be formulated as suppositories, eg containing conventional suppository bases such as cocoa butter or other glycerides.

For buccal administration the compositions may take the form of tablets or lozenges formulated in conventional manner.

For topical administration the compounds according to the invention may be formulated as ointments, creams, lotions, powders, pessaries, sprays, aerosols or drops (eg eye or nose drops). Ointments and creams may, for example, be formulated with an aqueous or oily base with the addition of suitable thickening and/or gelling agents. Such bases may thus, for example, include water and/or an oil such as liquid paraffin or a vegetable oil such as arachis oil or castor oil. Lotions may be formulated with an aqueous or oily base and will in general also contain one or more emulsifying agents, stabilising agents, dispersing agents, suspending agents, thickening agents and/or colouring agents. Powders may be formed with the aid of any suitable powder base, for example talc, lactose or starch. Drops may be formulated with an aqueous or non-aqueous base also comprising one or more dispersing agents, solubilising agents or suspending agents. Aerosol sprays are conveniently delivered from pressurised packs, with the use of a suitable propellant, eg dichlorodifluoromethane, trichlorofluoromethane, dichlorotetrafluoroethane, carbon dioxide or other suitable gas.

The compositions may contain 0.1-100% of the active material.

It may be possible to target a compound of the invention to a tumour by including in the composition specialised drug carrier systems such as liposomes, albumen microspheres or monoclonal antibodies.

For systemic administration the daily dosage as employed for adult human treatment will generally be within the range of from 5 mg to 5000 mg, preferably 50 mg to 2000mg, which may be administered in 1 to 5 divided daily doses, for example, depending on the route of administration and the condition of the patient. When the compositions comprise dosage units, each unit will preferably contain 10 mg to 2000 mg of active ingredient, for example 50 mg to 1000mg.

For topical administration the daily dosage as employed for adult human treatment will generally range from 0.1 mg to 1000mg, depending on the condition of the patient.

Suitable methods for preparing compounds of formula (I) are described below; the group X is as defined above except where otherwise indicated. It will be appreciated that the following reactions may require the use of, or conveniently may be applied to, starting materials having protected functional groups, and deprotection might thus be required as an intermediate or final step to yield the desired compound. Protection and deprotection of functional groups may be effected using conventional means. Thus, for example, amino groups may be protected by a group selected from aralkyl (e.g. benzyl), acyl or aryl (e.g. 2,4-dinitrophenyl); subsequent removal of the protecting group being effected when desired by hydrolysis or hydrogenolysis as appropriate using standard conditions. Hydroxyl groups may be protected using any conventional hydroxyl protecting group, for example, as described in 'Protective Groups in Organic Chemistry', Ed. J. F. W. McOmie (Plenum Press, 1973) or 'Protective Groups in Organic Synthesis' by Theodora W. Greene (John Wiley and Sons, 1981). Examples of suitable hydroxyl protecting groups include groups selected from alkyl (e.g. methyl, t-butyl or methoxymethyl), aralkyl (e.g. benzyl, diphenylmethyl or triphenylmethyl), heterocyclic groups such as tetrahydropyranyl, acyl (e.g. acetyl or benzoyl) and silyl groups such as trialkylsilyl (e.g. t-butyldimethylsilyl). The hydroxyl protecting groups may be removed by conventional techniques. Thus, for example, alkyl, silyl, acyl and heterocyclic groups may be removed by solvolysis, e.g. by hydrolysis under acidic or basic conditions. Aralkyl groups such as triphenylmethyl may similarly be removed by solvolysis, e.g. by hydrolysis under acidic conditions. Aralkyl groups such as benzyl may be cleaved by hydrogenolysis in the presence of a noble metal catalyst such as palladium-on-charcoal. Silyl groups may also conveniently be removed using a source of fluoride ions such as tetra-n-butylammonium fluoride.

According to another aspect of the present invention therefore, we provide processes for the preparation of compounds of formula (I) and their pharmaceutically acceptable derivatives. Thus, in a first process (A), compounds of formula (I) and their pharmaceutically acceptable derivatives may be prepared by reacting a compound of formula (II)

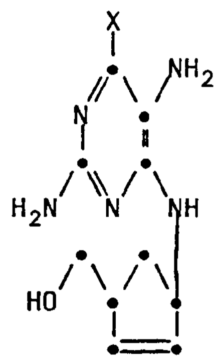

(II)

(where X is a substituent having the meaning in formula (I) above or is a protected form thereof and the 2-position amino group on the pyrimidine ring and/or the hydroxyl group in the cyclopentenylcarbinol grouping may be in protected form) or a pharmaceutically acceptable derivative thereof with nitrous acid, followed, where necessary, by removal of any protecting groups present.

The reaction with nitrous acid may be conveniently effected by adding a solution of a nitrite (e.g. sodium nitrite) to an aqueous solution of a compound of formula (II) in the presence of an acid (e.g. an organic acid such as acetic acid) conveniently at a low temperature (e.g. 0° to 10°C).

In another process (B), compounds of formula (I) and their pharmaceutically acceptable derivatives may be prepared by subjecting a compound of formula (I) (or a protected form thereof) or a pharmaceutically acceptable derivative thereof to an interconversion reaction whereby the substituent X initially present is replaced by a different substituent X.

Process (B) may conveniently be effected using a compound of formula (I) in which the substituent X is initially a halogen (e.g. chlorine) atom.

In one embodiment of process (B), compounds of formula (I) in which X represents a group $NRR^1$ (where R and $R^1$ are as defined previously) may be prepared by amination of a corresponding compound of formula (I) in which X represents a halogen atom (e.g. chlorine). The amination may be effected by reaction with a reagent $HNRR^1$ (where R and $R^1$ are as defined previously) conveniently in a solvent such as an alcohol (e.g. methanol). The reaction may be carried out at any suitable temperature and conveniently at an elevated temperature such as under reflux or, when liquid ammonia is used, in a sealed tube at about 50 to 80°C. Suitable conditions for the conversion of halides to secondary and tertiary amines have also been described by I. T. Harrison et. al., Compendium of Organic Synthetic Methods, Wiley-Interscience, New York (1971) at pages 250-252.

In another embodiment of process (B), compounds of formula (I) in which X represents a group OR (where R is as defined previously) may be prepared by displacement of the halogen (e.g. chlorine) atom with an appropriate anion $RO^-$. When R represents a hydrogen atom the displacement reaction may be carried out by hydrolysis which may be effected in water or in a mixture of water and a water-miscible solvent such as an alcohol (e.g. methanol or ethanol), an ether (e.g. dioxan or tetrahydrofuran), a ketone (e.g. acetone), an amide (e.g. dimethylformamide) or a sulphoxide (eg. dimethylsulphoxide), conveniently in the presence of an acid or base. Suitable acids include organic acids such as p-toluenesulphonic acid and inorganic acids such as hydrochloric, nitric or sulphuric acid. Suitable bases include inorganic bases such as alkali metal hydroxides or carbonates (e.g. sodium or potassium hydroxide or carbonate). Aqueous acid or base may also be used as the reaction solvent. The hydrolysis may conveniently be effected at a temperature in the range $^-10°$ to $^+150°C$, e.g. at reflux. When R represents a $C_{1-4}$alkyl or aryl group the anion $RO^-$ is conveniently formed from a corresponding alcohol ROH using an inorganic base such as an alkali metal (e.g. sodium metal) or an alkali metal hydride (e.g. sodium hydride). The reaction with the in situ formed anion may conveniently be effected at ambient temperature.

In a further embodiment of process (B), compounds of formula (I) in which X represents a group SH may be prepared by reacting a compound of formula (I) in which X is halogen (e.g. chlorine) with thiourea in a suitable solvent such as an alcohol (e.g. n-propanol) at an elevated temperature (e.g. reflux) followed by alkaline hydrolysis. Suitable bases which may be used include alkali metal hydroxides (e.g. sodium hydroxide). The reaction may conveniently be carried out according to the method of G. G. Urquart et. al. Org. Syn. Coll. Vol. 3, 363(1953) eg by refluxing the intermediate product with aqueous NaoH for about 0.25 to about 5 hours.

In another embodiment of process (B), compounds of formula (I) in which X represents a hydrogen atom may be prepared by reducing a compound of formula (I) in which X is halogen (e.g. chlorine) using a reducing

system which will not affect the rest of the molecule. Suitable reducing agents which may be used to effect the desired dehalogenation reaction include zinc metal/water using the method described by J. R. Marshall et. al., J. Chem. Soc., 1004 (1951). Alternatively, the reaction may be effected by photolysis in a suitable solvent such as tetrahydrofuran containing 10% triethylamine and conveniently in a Rayonet photochemical reactor (2537A) according to the method of V. Nair et. al., J. Org. Chem., 52, 1344 (1987).

In a yet further embodiment of process (B), compounds of formula (I) in which X represents a halogen atom may be prepared from a compound of formula (I) in which X is a different halogen atom by conventional methods of halide-halide exchange. Alternatively, when X is chlorine this substituent may be replaced by other halogen atoms by using various p-(halo)benzene diazonium chlorides according to well-known procedures.

Compounds of formula (I) in which X represents a group SR where R is a $C_{1-4}$alkyl or aryl group may be prepared from the corresponding thiols using standard methods of alkylation or arylation for example as described in US Patent NO. 4,383,114.

Many of the reactions described hereinabove have been extensively reported in the context of nucleoside synthesis, for example in Nucleoside Analogs - Chemistry, Biology and Medical Applications, R. T. Walker et. al., eds, Plenum Pless, New York (1979) at pages 193-223, the disclosure of which is incorporated by reference herein.

Pharmaceutically acceptable salts of the compounds of the invention may be prepared as described in US Patent No. 4,383,114, the disclosure of which is incorporated by reference herein. Thus, for example, when it is desired to prepare an acid addition salt of a compound of formula (I) the product of any of the above procedures may be converted into a salt by treatment of the resulting free base with a suitable acid using conventional methods. Pharmaceutically acceptable acid addition salts may be prepared by reacting the free base with an appropriate acid optionally in the presence of a suitable solvent such as an ester (e.g. ethyl acetate) or an alcohol (e.g. methanol, ethanol or isopropanol). Inorganic basic salts may be prepared by reacting the free base with a suitable base such as an alkoxide (e.g. sodium methoxide) optionally in the presence of a solvent such as an alcohol (e.g. methanol). Pharmaceutically acceptable salts may also be prepared from other salts, including other pharmaceutically acceptable salts, of the compounds of formula (I) using conventional methods.

A compound of formula (I) may be converted into a pharmaceutically acceptable phosphate or other ester by reaction with a phosphorylating agent, such as $POCl_3$ or a suitable esterifying agent, such as an acid halide or anhydride, as appropriate. An ester or salt of a compound of formula (I) may be converted to the parent compound for example by hydrolysis.

The compounds of formula (II) may be prepared from the compound of formula 2a

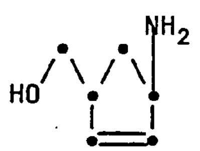

2a

by reaction with an excess of a pyrimidine of formula (III)

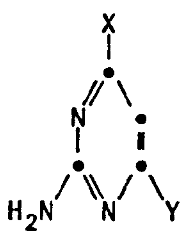

(III)

(wherein X is as defined in formula (I) above and Y is a halogen atom, e.g. chlorine) in the presence of an amine base such as triethylamine and in an alcoholic solvent (e.g. n-butanol) conveniently at reflux to give a compound of formula (IV)

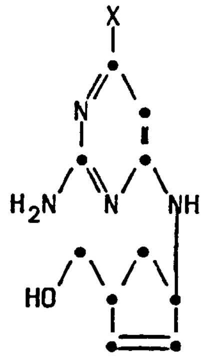

(IV)

which may be diazotized using a diazonium salt $ArN_2^+E^-$ (wherein Ar represents an aromatic group, e.g. p-chlorophenyl, and $E^-$ represents an anion, e.g. a halide such as chloride) in a solvent such as water, an organic acid such as acetic acid or a mixture thereof, conveniently at about ambient temperature to give a compound of formula (V)

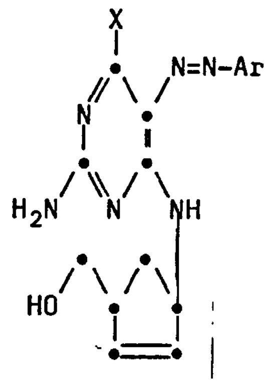

(V)

(wherein Ar is as defined just above) which may be converted to the desired compound of formula (II) by reduction using for example a reducing metal such as zinc in the presence of an acid, e.g. acetic acid. It will be appreciated that the choice of reducing agent will depend on the nature of the group X.

The compound 2a may be prepared from the versatile precursor, 1α-acetylamino-3α-acetoxy-methylcyclopent-2-ene (1a) by hydrolysis in the presence of a mild base, such as an alkaline earth metal hydroxide.

In a particularly convenient process embodiment compounds of formula (I) may be prepared according to the synthesis outlined below:

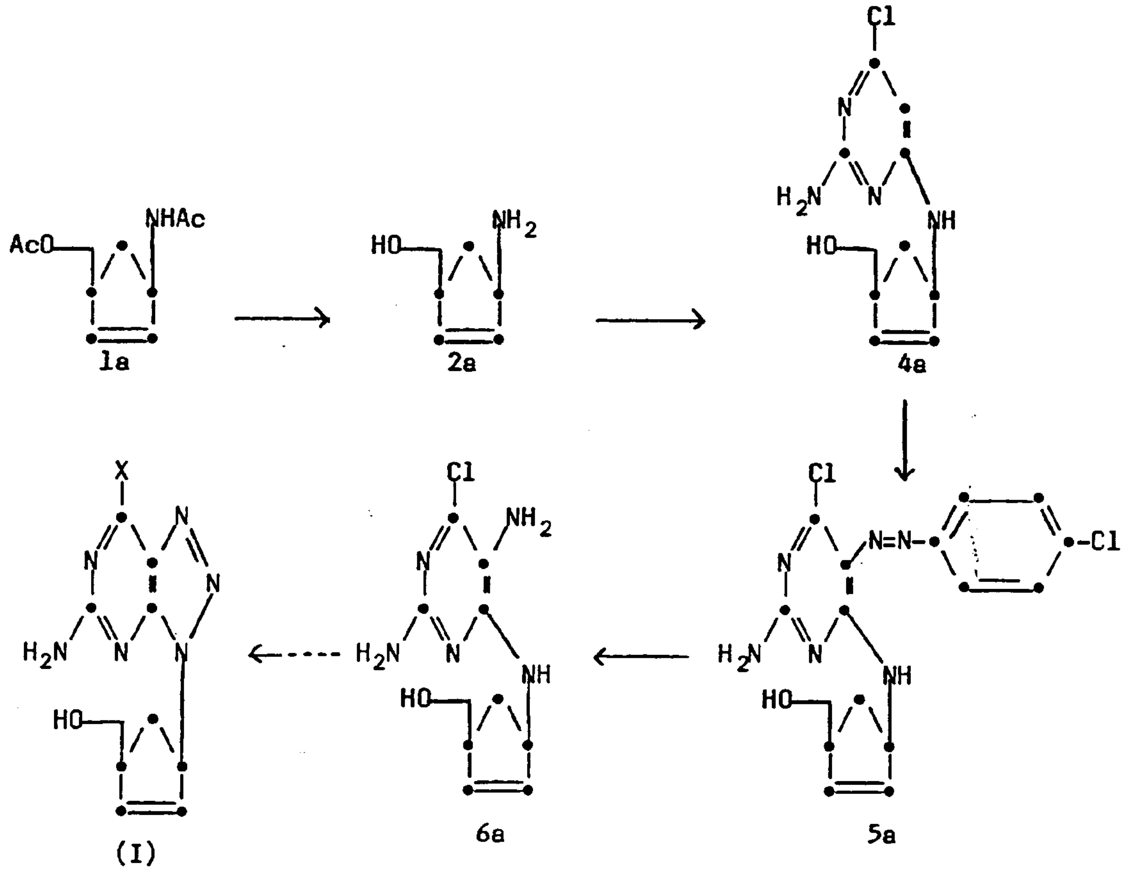

1a 2a 4a

(I) 6a 5a

The compound 1a is a known compound described in US Patent No. 4,138,562.

Where the compound of formula (I) is desired as a single isomer it may be obtained either by resolution of the final product or by stereospecific synthesis from isomerically pure starting material or any convenient intermediate.

Resolution of the final product, or an intermediate or starting material therefor may be effected by any suitable method known in the art : see for example 'Stereochemistry of Carbon Compounds' by E. L. Eliel (McGraw Hill, 1962) and 'Tables of Resolving Agents' by S. H. Wilen.

One convenient method for obtaining chirally pure compounds of formula (I) is by enzymatic conversion of a racemic mixture of the compound or a precursor thereof. By such a method both (+) and (-) compounds of formula (I) may be obtained in optically pure form. Suitable enzymes include deaminases such as adenosine deaminase.

The invention will be further described by reference to the following detailed examples wherein elemental analyses were performed by M-H-W Laboratories, Phoenix, AZ. Melting points were determined on a Mel-Temp apparatus and are corrected. Nuclear magnetic resonance spectra were obtained on Jeol FX 90QFT or Nicollet NT300 spectrometers and were recorded in DMSO-$D_6$. Chemical shifts were expressed in ppm down-field from $Me_4Si$. IR spectra were determined as KBr pellets with a Nicollet 50XC FT-IR spectrometer, and UV spectra were determined on a Beckmann DU-8 spectrophotometer. Mass spectra were obtained with an AEI Scientific Apparatus Limited MS-30 mass spectrometer. Thin layer chromatography (TLC) was performed on 0.25mm layers of Merck silica gel (230-400 mesh). All chemicals and solvents are reagent grade

Intermediate 1

(±)-(1α,4α)-4-[(2-Amino-6-chloro-4-pyrimidinyl)-amino]-2-cyclopentenylcarbonil (4a)

A mixture of 1α-acetylamino-3α-acetoxymethyl cyclopent-2-ene (1a) (3.0g, 15 mmol) and aqueous barium hydroxide (0.5N, 300ml) was refluxed overnight. After cooling, it was neutralized with dry ice. The precipitate was filtered out, and the aqueous solution was concentrated to dryness. The residue was extracted with absolute ethanol and concentrated again to yield 2a as a colourless syrup 1.6g (14mmol).

To 14 mmol of crude 2a, 2-amino-4,6-dichloro-pyrimidine (3.74g, 22.8 mmol), triethylamine (15ml) and n-butanol (75ml) were added and the mixture was refluxed for 48 hr. The volatile solvents were removed, residue was treated with methanol to separate the undissolved by- product (the double pyrimidine nucleoside). The methanol solution was absorbed on silica gel (8g) packed into a column (4.0 x 14cm) and eluted with $CHCl_3$-MeOH (40:1) to yield 1.52g (42%) of crude 4a. The product was recrystallised from ethyl acetate to yield 4a; m.p. 132-134°C.

Intermediate 2

(±)-(1α,4α)-4-([(2-Amino-6-chloro-5-(4-chlorophenyl)-azo]-4-pyrimidinyl-amino)-2-cyclopentenylcarbinol (5a)

A cold diazonium salt solution was prepared from p-chloroaniline (1.47g, 11.5 mmol) in 3N HCl (25ml) and sodium nitrite (870mg, 12.5 mmol) in water (10ml). This solution was added to a mixture of 4a (2.40g, 10 mmol), acetic acid (50ml), water (50ml) and sodium acetate trihydrate (20g). The reaction mixture was stirred overnight at room temperature. The yellow precipitate was filtered and washed with cold water until neutral, then it was air-dried in the fumehood to yield 3.60g (94%), of 5a, m.p. 229°C (dec). The analytical sample was obtained from acetone-methanol (1:2), m.p. 241-243°C (dec).

Intermediate 3

(±)-(1α,4α)-4-[(2,5-Diamino-6-chloro-4-pyrimidinyl)-amino]-2-cyclopentenylcarbinol (6a)

A mixture of 5a (379mg, 1 mmol), zinc dust (0.65g, 10 mmol), acetic acid (0.32 ml), water (15ml) and ethanol (15ml) was refluxed under nitrogen for 3 hr. The zinc was removed and the solvents were evaporated. The residue was absorbed on silica gel (2g), packed into a column (2.0 x 18cm), and eluted with $CHCl_3$-MeOH (15:1). A pink syrup was obtained. Further purification from methanol-ether yielded 6a as pink crystals, 170mg (66%), m.p. 168-170°C.

Example 1

(±)-(1α,4α)-4-(5-Amino-7-chloro-3H-1,2,3-triazolo[4,5-d]pyrimidin-3-yl)-2-cyclopentenyl carbinol

To a cold solution of 6a (225mg, 1mmol) in acetic acid (1.5ml) and water (2.5ml) was added sodium nitrite (83mg, 1.2 mmol) in water (2ml). The reaction was monitored by starch-potassium iodide paper. After stirring for 1hr at 0°C, the precipitate was filtered and washed with cold water, then dried over phosphorus pentoxide in vacuo to yield the title compound as off-white crystals, 218mg (81%). The crude product was recrystallised from methanol, m.p. 153-155°C (dec). MS (30 ev, 220°C): m/e 266 and 268 ($M^+$ and $M^+ + 2$), 236 ($M^+$ -30), 170 ($B^+$); IR: 3600-3000 ($NH_2$, OH), 1650,1600 (C=C, C=N).

Example 2

(±)-(1α,4α)-4-(5-Amino-7-hydroxy-3H-1,2,3-triazolo[4,5-d]pyrimidin-3-yl)-2-cyclopentenyl carbinol

A mixture of the product of Example 1 (218mg, 0.8 mmol) and aqueous sodium hydroxide (0.25N, 10ml) was refluxed for 3 hr, then was adjusted to pH 3 with 6N hydrochloric acid. The gelatinous precipitate was filtered and washed with cold water. It was dried over phosphorus pentoxide in vacuo to yield the title compound as an off-white solid, 181mg (90%), m.p. 222-224°C (dec). After recrystallisation from water, the m.p. was 223-225°C (dec). MS (20 ev, 300°C): m/e 248 ($M^+$), 217 ($M^+$ -31), 152 ($B^+$); IR: 3600-3000 ($NH_2$, OH), 1750,1600 (C=C, C=N).

Example 3

(±)-(1α,4α)-4-(5,7-Diamino-3H-1,2,3-triazolo[4,5-d]pyrimidin-3-yl)-2-cyclopentenyl carbinol

Liquid ammonia was passed into a solution of the product of Example 1 (267mg, 1mmol) in methanol (10ml) at -80°C in a bomb. The bomb was sealed and heated at 60°C for 20hr. Ammonia and methanol were evaporated. The residual mixture was absorbed on silica gel (2g), packed in a column (2.0 x 10cm) and eluted by $CHCl_3$-MeOH (15:1) to yield the title compound as white crystals, 204mg (83%). The crude product was recrystallised from ethanol-water (2:1), to yield the title compound of m.p. 240-242°C (dec). MS (30 ev, 240°C): m/e 247 ($M^+$), 229 ($M^+$-18),217 ($M^+$-30), 151 ($B^+$); IR: 3600-3100 ($NH_2$, OH), 1700,1650,1600 (C=O, C=C, C=N).

Example 4

Cytotoxicity Assay

The $ED_{50}$ cytotoxicity concentrations determined for the compounds of Examples 1 and 2 in the P-388 mouse leukemia cell culture assay are given in Table I.

Table I - Inhibitory Concentrations of Carbocyclic Nucleosides for P-388 Leukemia Cells in Cultures *

| Example Number | $ED_{50}$ in µg/ml |
| --- | --- |
| 1 | 1.0 |
| 2 | 4.5 |

* Assay Technique : R. G. Almquist and R. Vince, J. Med. Chem., 16, 1396 (1973).

Therefore, both compounds listed in Table I are active against P-388 mouse leukemia.

The following are examples of pharmaceutical compositions according to the invention. The term 'Active Ingredient' as used hereinafter means a compound of the invention.

Example A-Dry Powder for Injection

| | mg/vial |
| --- | --- |
| Active ingredient | equivalent to 100mg acid |
| Trisodium citrate | 8.8 |
| Citric acid | 0.2 |

Method 1

Blend the sterile ingredients until homogeneous. Fill aseptically into glass vials. Purge the headspace with nitrogen and close the vials using rubber closures and metal overseals.

Method 2

Dissolve the ingredients in water for injections B.P. Sterilise the solution by membrane filtration. Aseptically fill into freeze-drying vials and place suitable rubber freeze-drying closures in position. Freeze-dry, filling the vials with nitrogen at the end of the cycle. Fully insert the closures and apply metal overseals.

Constitution

Constitute with a suitable sterile vehicle, e.g. using water for injections or using a 5% w/v dextrose solution, as an injection (e.g. in a 10ml volume) or an infusion (e.g. in a 100ml volume).

Example B - Oral Tablet

| | mg/tablet |
|---|---|
| Active Ingredient | equivalent to 250mg acid |
| Microcrystalline cellulose | 231 |
| Sodium starch glycolate | 6 |
| Magnesium stearate | 2 |

Sieve the ingredients and blend until homogeneous. Compress with appropriate punches. The tablets may be covered with a thin polymer coat applied by the usual film coating techniques. A pigment may be included in the film coat.

## Claims

**Claims for the following Contracting States : AT, BE, CH, DE, FR, GB, IT, LI, LU, NL, SE**

1. Compounds of formula (I)

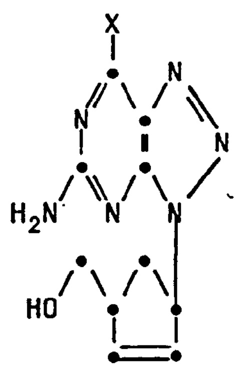

(I)

wherein X is hydrogen, $NRR^1$, SR, OR or halogen;
R and $R^1$ may be the same or different and are selected from hydrogen, $C_{1-4}$alkyl and aryl;
and pharmaceutically acceptable derivatives thereof.

2. Compounds of formula (I) as claimed in Claim 1 in which X represents $NRR^1$, OR or halogen.

3. A compound of formula (I) as claimed in Claim 1 in which X represents $NH_2$.

4. A compound of formula (I) as claimed in Claim 1 in which X represents OH.

5. A compound of formula (I) as claimed in Claim 1 in which X represents chlorine.

6. A process for the preparation of compounds of formula (I) (as defined in Claim 1) and pharmaceutically acceptable derivatives thereof, which comprises at least one of the following steps :
(A) reacting a compound of formula (II)

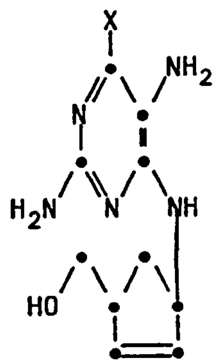

(II)

(where X is a substituent having the meaning in Claim 1 or is a protected form thereof and the 2-position amino group on the pyrimidine ring and/or the hydroxyl group in the cyclopentenylcarbinol grouping may be in protected form) or a pharmaceutically acceptable derivative thereof with nitrous acid, followed where necessary, by removal of any protecting groups present;

(B) subjecting a compound of formula (I) (or a protected derivative thereof) or a pharmaceutically acceptable derivative thereof to an interconversion reaction whereby the substituent X initially present is replaced by a different substituent X.

(C) converting a free compound of formula (I) into a pharmaceutically acceptable derivative thereof.

7. A process as claimed in Claim 6 wherein process step (B) is effected using a compound of formula (I) in which the substituent X is initially a halogen atom.

8. A compound of formula (I) as claimed in any one of Claims 1 to 5 or a pharmaceutically acceptable derivative thereof for use in therapy.

9. A compound of formula (I) as claimed in any one of Claims 1 to 5 or a pharmaceutically acceptable derivative thereof for use in the treatment of the human or non-human animal body to combat cancers therein.

10. A pharmaceutical composition comprising as an active ingredient a compound of formula (I) (as defined in Claim 1) or a pharmaceutically acceptable derivative thereof together with one or more pharmaceutical carriers or excipients.

**Claims for the following Contracting States : ES, GR**

1. A process for the preparation of compounds of formula (I)

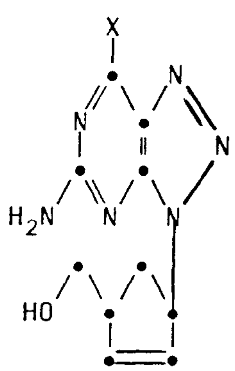

(I)

wherein X is hydrogen, NRR$^1$, SR, OR or halogen;

R and R$^1$ may be the same or different and are selected from hydrogen, $C_{1-4}$alkyl and aryl; and pharmaceutically acceptable derivatives thereof, said process comprising

(A) reacting a compound of formula (II)

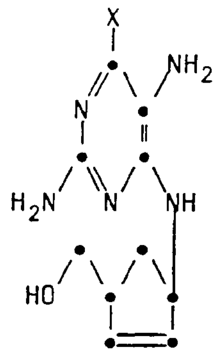

(II)

(where X is a substituent having the meaning in formula (I) or is a protected form thereof and the 2-position amino group on the pyrimidine ring and/or the hydroxyl group in the cyclopentenylcarbinol grouping may be in protected form) or a pharmaceutically acceptable derivative thereof with nitrous acid, followed where necessary, by removal of any protecting groups present;
(B) subjecting a compound of formula (I) (or a protected derivative thereof) or a pharmaceutically acceptable derivative thereof to an interconversion reaction whereby the substituent X initially present is replaced by a different substituent X; or
(C) converting a free compound of formula (I) into a pharmaceutically acceptable derivative thereof.

2. A process as claimed in Claim 1 wherein process step (B) is effected using a compound of formula (I) in which the substituent X is initially a halogen atom.

3. A process as claimed in Claim 1 or Claim 2 being a process for the preparation of a compound of formula (I) in which X represents $NRR^1$, OR or halogen.

4. A process as claimed in Claim 1 or Claim 2 being a process for the preparation of a compound of formula (I) in which X represents $NH_2$.

5. A process as claimed in Claim 1 or Claim 2 being a process for the preparation of a compound of formula (I) in which X represents OH.

6. A process as claimed in Claim 1 or Claim 2 being a process for the preparation of a compound of formula (I) in which X represents chlorine.

## Patentansprüche

### Patentansprüche für folgende Vertragsstaaten : AT, BE, CH, DE, FR, GB, IT, LI, LU, NL, SE

1. Verbindungen der Formel (I)

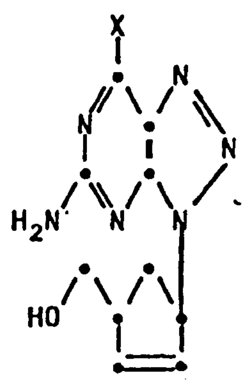

(I)

in der X Wasserstoff, $NRR^1$, SR, OR oder Halogen ist,

R und $R^1$ gleich oder verschieden sein können und aus der Gruppe Wasserstoff, $C_{1-4}$-Alkyl und Aryl ausgewählt sind,
sowie ihre pharmazeutisch annehmbaren Derivate.

2. Verbindungen der Formel (I) nach Anspruch 1, in der X $NRR^1$, OR oder Halogen bedeutet.

3. Verbindung der Formel (I) nach Anspruch 1, in der X $NH_2$ bedeutet.

4. Verbindung der Formel (I) nach Anspruch 1, in der X OH bedeutet.

5. Verbindung der Formel (I) nach Anspruch 1, in der X Chlor bedeutet.

6. Verfahren zur Herstellung von Verbindungen der Formel (I) (wie in Anspruch 1 definiert) und ihrer pharmazeutisch annehmbaren Derivate, das wenigstens einen der folgenden Schritte umfaßt:
(A) Umsetzung einer Verbindung der Formel (II)

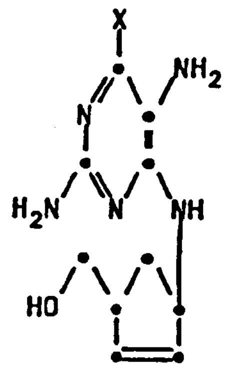

(II)

(in der X ein Substituent entsprechend der Definition in Anspruch 1 oder eine geschützte Form eines solchen ist und die Aminogruppe in der 2-Stellung der Pyrimidinrings und/oder die Hydroxylgruppe in der Cyclopentenylhydroxymethylgruppe geschützt sein können) oder eines pharmazeutisch annehmbaren Derivats davon mit salpetriger Säure, wobei ggf. anwesende Schutzgruppen anschließend entfernt werden;
(B) Umwandlung einer Verbindung der Formel (I) (oder eines geschützten Derivats einer solchen) oder eines pharmazeutisch annehmbaren Derivats einer solchen, durch die der ursprünglich vorhandene Substituent X durch einen anderen Substituenten X ersetzt wird;
(C) Umwandlung einer freien Verbindung der Formel (I) in eines ihrer pharmazeutisch annehmbaren Derivate.

7. Verfahren nach Anspruch 6, bei dem der Verfarensschritt (B) unter Verwendung einer Verbindung der Formel (I) durchgeführt wird, in der der Substituent X ursprünglich ein Halogenatom ist.

8. Verbindung der Formel (I) nach einem der Ansprüche 1 bis 5 oder eines pharmazeutisch annehmbaren Derivats zur therapeutischen Verwendung.

9. Verbindung der Formel (I) nach einem der Ansprüche 1 bis 5 oder eines pharmazeutisch annehmbaren Derivats zur Verwendung bei der Behandlung des menschlichen oder nichtmenschlichen, tierischen Körpers zur Bekämpfung von Krebs.

10. Pharmazeutische Zusammensetzung, enthaltend als aktiven Wirkstoff eine Verbindung der Formel (I) (wie in Anspruch 1 definiert) oder ein pharmazeutisch annehmbares Derivat zusammen mit einem oder mehreren pharmazeutischen Trägern oder Bindemitteln.

**Patentansprüche für folgende Vertragsstaaten : ES, GR**

1. Verfahren zur Herstellung von Verbindungen der Formel (I)

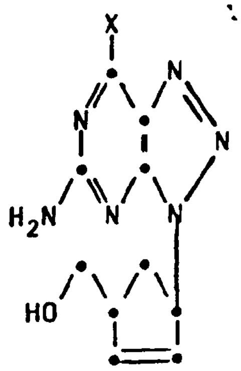

(I)

in der X Wasserstoff, $NRR^1$, SR, OR oder Halogen ist,
R und $R^1$ gleich oder verschieden sein können und aus der Gruppe Wasserstoff, $C_{1-4}$-Alkyl und Aryl ausgewählt sind, sowie ihrer pharmazeutisch annehmbaren Derivate,
unfassend

(A) Umsetzung einer Verbindung der Formel(II)

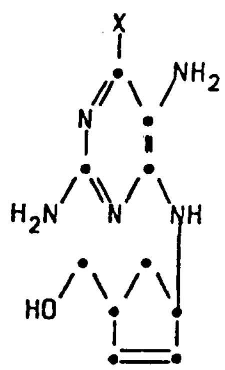

(II)

(in der X ein Substituent entsprechend der Definition in Formel 1 oder eine geschützte Form eines solchen ist und die Aminogruppe in der 2-Stellung des Pyrimidinrings und/oder die Hydroxylgruppe in der Cyclopentenylhydroxymethylgruppe geschützt sein können), oder eines pharmazeutisch annehmbaren Derivats davon mit salpetriger Säure, wobei ggf. anwesende Schutzgruppen anschließend entfernt werden;

(B)Umwandlung einer Verbindung der Formel (I) (oder eines geschützten Derivats einer solchen) oder eines pharmazeutisch annehmbaren Derivats einer solchen, durch die der ursprünglich vorhandene Substituent X durch einen anderen Substituenten X ersetzt wird; oder

(C)Umwandlung einer freien Verbindung der Formel (I) in eines ihrer pharmazeutisch annehmbaren Derivate.

2. Verfahren nach Anspruch 1, bei dem der Verfahrensschritt (B) unter Verwendung einer Verbindung der Formel (I) durchgeführt wird, in der der Substituent X ursprünglich ein Halogenatom ist.

3. Verfahren nach Anspruch 1 oder 2 zur Herstellung einer Verbindung der Formel (I), in der X $NRR^1$, OR oder Halogen bedeutet.

4. Verfahren nach Anspruch 1 oder 2 zur Herstellung einer Verbindung der Formel (I), in der X $NH_2$ bedeutet.

5. Verfahren nach Anspruch 1 oder 2 zur Herstellung einer Verbindung der Formel (I), in der X OH bedeutet.

6. Verfahren nach Anspruch 1 oder 2 zur Herstellung einer Verbindung der Formel (I), in der X Chlor bedeutet.

## Revendications

### Revendications pour les Etats contractants suivants : AT, BE, CH, DE, FR, GB, IT, LI, LU, NL, SE

1. Composés de formule (I)

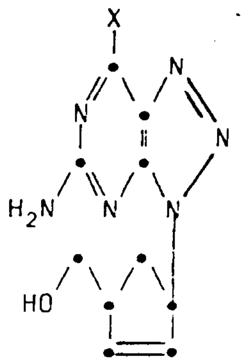

(I)

dans laquelle X est un hydrogène, $NRR^1$, SR, OR ou un halogène ;

R et $R^1$ peuvent être semblables ou différents et sont choisis parmi un hydrogène, un alcoyle en $C_{1-4}$ et un aryle ;

et leurs dérivés pharmaceutiquement acceptables.

2. Composés de formule (I) selon la revendication 1, où X représente $NRR^1$, OR ou un halogène.

3. Un composé de formule (I) selon la revendication 1, où X représente $NH_2$.

4. Un composé de formule (I) selon la revendication 1, où X représente OH.

5. Un composé de formule (I) selon la revendication 1, où X représente le chlore.

6. Un procédé pour la préparation de composés de formule (I) (comme définis dans la revendication 1) et de leurs dérivés pharmaceutiquement acceptables, qui comprend au moins une des étapes suivantes :

(A) faire réagir un composé de formule (II)

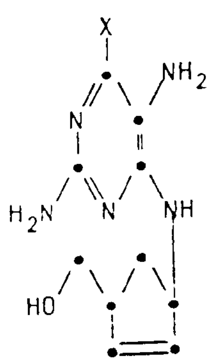

(II)

(dans laquelle X est un substituant ayant la même signification que dans la revendication 1 ou une forme protégée de celui-ci et le groupe amino de la position 2 sur le cycle pyrimidine et/ou le groupe hydroxyle dans le groupe cyclopenténylcarbinol peuvent être sous une forme protégée) ou un dérivé pharmaceutiquement acceptable de celui-ci, avec l'acide nitreux, puis, le cas échéant, éliminer tout groupe protecteur présent ;

(B) soumettre un composé de formule (I) (ou un dérivé protégé de celui-ci) ou un dérivé pharmaceutiquement acceptable de celui-ci, à une réaction d'interconversion pour que le substituant X initialement présent soit remplacé par un substituant X différent ;

(C) transformer un composé libre de formule (I) en un de ses dérivés pharmaceutiquement acceptables.

7. Un procédé selon la revendication 6, dans lequel l'étape opératoire (B) est effectuée par emploi d'un composé de formule (I) dans laquelle le substituant X est initialement un atome d'halogène.

8. Un composé de formule (I) selon l'une quelconque des revendications 1 à 5 ou un dérivé pharmaceutiquement acceptable de celui-ci pour l'emploi en thérapeutique.

9. Un composé de formule (I) selon l'une quelconque des revendications 1 à 5 ou un dérivé pharmaceutiquement acceptable de celui-ci pour l'emploi dans le traitement de l'organisme de l'homme ou d'un animal autre que l'homme pour lutter contre des cancers.

10. Une composition pharmaceutique comprenant comme ingrédient actif un composé de formule (I) (comme défini dans la revendication 1) ou un dérivé pharmaceutiquement acceptable de celui-ci avec un ou plusieurs véhicules ou excipients pharmaceutiques.

**Revendications pour les Etats contractants suivants : ES, GR**

1. Un procédé pour la préparation de composés de formule (I)

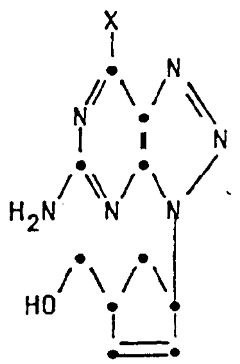

(I)

dans laquelle X est un hydrogène, NRR[1], SR, OR ou un halogène ;

R et R[1] peuvent être semblables ou différents et sont choisis parmi un hydrogène, un alcoyle en $C_{1-4}$ et un aryle ;

et de leurs dérivés pharmaceutiquement acceptables, ledit procédé comprenant :

(A) faire réagir un composé de formule (II)

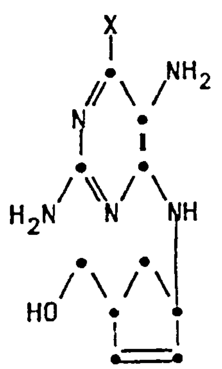

(II)

(dans laquelle X est un substituant ayant la même signification que dans la formule 1 ou une forme protégée de celui-ci et le groupe amino de la position 2 sur le cycle pyrimidine et/ou le groupe hydroxyle dans le groupe cyclopenténylcarbinol peuvent être sous une forme protégée) ou un dérivé pharmaceutiquement acceptable de celui-ci, avec l'acide nitreux, puis, le cas échéant, éliminer tout groupe protecteur présent ;

(B) soumettre un composé de formule (I) (ou un dérivé protégé de celui-ci) ou un dérivé pharmaceutiquement acceptable de celui-ci, à une réaction d'interconversion pour que le substituant X initialement présent soit remplacé par un substituant X différent ; ou
(C) transformer un composé libre de formule (I) en un de ses dérivés pharmaceutiquement acceptables.

**2.** Un procédé selon la revendication 1, dans lequel l'étape opératoire (B) est effectuée par emploi d'un composé de formule (I) dans laquelle le substituant X est initialement un atome d'halogène.

**3.** Un procédé selon la revendication 1 ou la revendication 2, qui est un procédé pour la préparation d'un composé de formule (I) dans laquelle X représente $NRR^1$, OR ou un halogène.

**4.** Un procédé selon la revendication 1 ou la revendication 2, qui est un procédé pour la préparation d'un composé de formule (I) dans laquelle X représente $NH_2$.

**5.** Un procédé selon la revendication 1 ou la revendication 2, qui est un procédé pour la préparation d'un composé de formule (I) dans laquelle X représente OH.

**6.** Un procédé selon la revendication 1 ou la revendication 2, qui est un procédé pour la préparation d'un composé de formule (I) dans laquelle X représente le chlore.